# EUROPEAN PATENT APPLICATION

(11) **EP 1 224 932 A1**
(43) Date of publication of application: **24.07.2002**
(21) Application number: 00953455.3
(22) Date of filing: 17.08.2000
(51) Int. Cl.: A61K 31/404, A01N 1/02, A61P 25/28, A61P 21/00, A61P 25/16, A61P 25/14, A61P 27/02, A61P 9/10, A61P 9/04, A61P 1/16, A61P 13/12, A61P 43/00, C07D 403/04

(54) **DRUGS INHIBITING CELL DEATH**

(30) Priority: 20.08.1999 JP 23346599
(71) Applicant: SAGAMI CHEMICAL RESEARCH CENTER, Sagamihara-shi, Kanagawa 229-0012 (JP); Asakai, Rei, Saitama-shi, Saitama 330-0801 (JP)
(72) Inventor: ASAKI, Rei, Saitama-shi, Saitama 330-0801 (JP); SODEOKA, Mikiko, Sendai-shi, Miyagi 980-8577 (JP); FUJITA, Mikako, Tokushima-shi, Tokushima 770-0045 (JP); KATOH, Miho, Ebina-shi, Kanagawa 243-0414 (JP)
(74) Representative: Albrecht, Thomas, Dr.
(86) International application number: JP0005496
(87) International publication number: WO0113916

(57) **Abstract**

A cell death inhibitor, a pharmaceutical or a preservative for organs, tissues or cells, a comprising, as an active ingredient, an indolylmaleimide derivative represented by the following formula (I), which is useful for inhibiting death of cells, the drug being expected as a preventive or a remedy for the progress of various diseases wherein cell death participates in progress and exacerbation thereof:

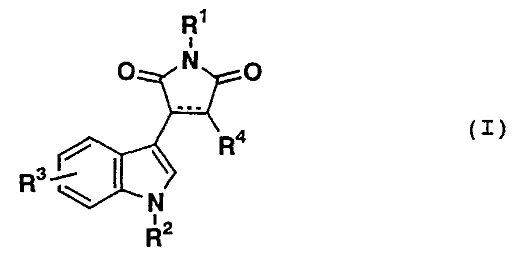

## Description

### Technical Field

The present invention relates to a cell death inhibitor capable of inhibiting cell death induced by various substances in living body or foreign stimulants, or stimuli such as temperature, radiation and so on; its use as drugs for treating neurodegenerative diseases, diseases of circulatory organs, hepatitis, renal diseases, inflammatory skin disorders, radiation disorders, viral diseases, prion diseases, functional deficiency of transplanted organs, or the like, or preventing progress of the symptoms of the diseases.

### Background of the Invention

Recent progress of the study as to cell death have revealed that cell death of cells essential for living body, particularly apoptosis is involved in progress and exacerbation of a variety of diseases *(Science,* Vol. 267, p. 1456, 1995). Apoptosis is a type of cell death in which cells commit a death using their own molecular machinery, characterized generally by (1) chromatin aggregation, (2) cell shrinkage, (3) blebbing of plasma membrane (formation of processes), (4) nuclear fragmentation, (5) formation of apoptotic bodies, (6) DNA fragmentation, and (7) phagocytosis (scavenging cell debris) by neighboring cells and macrophages. In contrast, there is another type of cell death, called necrosis, characterized by cell swelling and lysis, which occurs without executing the apoptotic processes when cells are exposed to excessive radiation, heat, noxious stimulants or the like. However, the cell death caused by the own molecular machinery does not always show a full set of the apoptosis characteristics described above, depending on species of cells, environments under which cells are present, and species and strength of cell death stimulants. Likewise, necrosis in view of pathology sometimes contains a cell death which some own molecular machinery is responsible for. In the invention, such cell death is also included in apoptosis.

Examples of the diseases whose progress and exacerbation are caused by apoptotic cell death are as follows: neurodegenerative diseases such as Alzheimer's disease (*Bio Science terminology library: apoptosis*/*separate volume of* "*Jikken Igaku (Experimental Medicine)", p.* 168, 1996), spinal muscular atrophy (SMA) (*Bio Science terminology library: apoptosis*/*separate volume of* "*Jikken Igaku (Experimental Medicine)",* p. 173, 1996), amyotrophic lateral screrosis (ALS) (*Bio Science terminology library: apoptosis*/*separate volume of* "*Jikken Igaku (Experimental Medicine)",* p. 176, 1996), Parkinson's disease (*J. Neurochem.,* Vol. 69, p. 1612, 1997), Huntington's disease (*J. Neurosci*., Vol. 15, p. 3775, 1995), pigmentary degeneration of the retina and glaucoma *(Bio Science terminology library: apoptosis*/*separate volume of* "*Jikken Igaku (Experimental Medicine)",* p. 196, 1996), cerebellar degeneration and neonatal jaundice *(Progress in Drug Research,* Vol. 48, p. 55, 1997); myasthenia gravis (*J*. *Clinical Investigation,* Vol. 99, p. 2745, 1997); brain ischemia from apoplexy and the like, and successive delayed neuronal death (DND) (*Bio Science terminology library: apoptosis*/*separate volume of* "*Jikken Igaku (Experimental Medicine)",* p. 180, p. 182, 1996), ischemic heart disease due to myocardial infarction (myocardial ischemia and disorder after reperfusion), viral myocarditis, autoimmune myocarditis (congestive cardiomyopathy and chronic myocarditis), myocardial disorders or death due to hypertrophic heart and heart failure, arrythmogenic right ventricular cardiomyopathy (*Bio Science terminology library: apoptosis*/*separate volume of* "*Jikken Igaku (Experimental Medicine)",* p. 198, 1996; *Kekkan to Naihi (Blood Vessel and Endothelium),* Vol. 7, *p*. 357, *p*. 364, *p*. 370, 1997); alcoholic hepatitis, viral hepatitis *(Bio Science terminology library: apoptosis*/*separate volume of* "*Jikken Igaku (Experimental Medicine)",* p. 190, 1996), renal diseases such as glomerulonephritis, hemolytic uremic syndrome and the like (*Bio Science terminology library:* *apoptosis*/*separate volume of* "*Jikken Igaku (Experimental Medicine*)", p. 192, 1996), acquired immunodeficiency syndrome (AIDS) (*Bio Science terminology library: apoptosis*/*separate volume of* "*Jikken Igaku (Experimental Medicine)",* p. 156, 1996; *Ketsueki, Meneki, Shuyou (Blood, Immunity, Cancer),* Vol.2, p. 432, 1997), inflammatory skin disorders such as toxic epidermal necrolysis (TEN) and multiform exudative erythema, alopecia, graft versus host disease (GVH) (*Bio Science terminology library: apoptosis*/*separate volume of* "*Jikken Igaku (Experimental Medicine)",* p. 194, 1996), radiation disorders *(Bio Science terminology library: apoptosis*/*separate volume of "Jikken Igaku (Experimental Medicine)", p.* 160, 1996), side effects due to anti-cancer drugs, anti-viral drugs and the like, disorders due to toxic agents such as sodium azide, potassium cyanide and the like (*Bio Science terminology library: apoptosis*/*separate volume of* "*Jikken Igaku (Experimental Medicine)",* p. 162, 1996), sepsis *(Critical Care Medicine,* Vol. 25, p. 1298, 1996), osteomyelo-dysplasia such as aplastic anemia and the like *(Leukemia,* Vol. 7, p. 144, 1993), insulin dependent diabetes (*Diabetes*, Vol. 44, p. 733, 1995), prion diseases such as Creutzfeldt-Jakob's disease (*J*. *Neural Transmission,* Supplementum, Vol. 50, p. 191, 1997), and so on. In organ transplantation, it has been suggested that apoptosis due to reactive oxygen species and various chemical mediators generated after reperfusion of anoxic organs by isolation or cardiac arrest of a donor is responsible for functional deficiency of transplanted organs (for example, *Ishoku (Transplantation),* Vol. 27, p. 15, 1992). Probably, rejection reaction after transplantation of an organ, tissues, or cells may be a result of apoptosis of the transplanted cells, which occurs when they are attacked by recipient immune cells. It is thus reasonably concluded that chemical compounds capable of inhibiting cell death can be a promising drug that heals these diseases effectively, or inhibits or stops progress and exacerbation of the symptoms of these diseases.

In the transplantation of organs or tissues, graft survival rate after transplantation depends on the preserving conditions of the organs or tissues isolated from a donor. Accordingly, it is expected to improve organ and tissue preservation by adding chemical compounds inhibiting cell death into preservation liquids for the organs and tissues. Unlike immortalized cells or cancer cells, primary cultured cells isolated from a living body are usually difficult to culture *in vitro.* For long time cultivation, appropriate concentration of additives including various growth factors are required in the culture medium depending on species of the cells, and apoptosis easily occurs in case that the culture conditions are improper. When cells are cultured for research or medical purposes, it is expected that addition of a chemical compound inhibiting cell death would lead successful cell cultivation.

Apoptosis is known to be triggered by a wide variety of physiological substances such as cytokines including interleukins, hormones including glucocorticoids, excitotoxic amino acids including glutamic acid and NMDA, and membrane proteins represented by Fas ligand, depending on cell types. It is also triggered by deprivation of a specific growth factor or the like in some cell types. There are common apoptosis triggers irrespective of cell type, such as reactive oxygen species generators including hydrogen peroxide and the like, NO generators including SNP and the like, heat, and radiation. A number of chemical compounds are also reported to be able to induce apoptosis. Recent studies have shown that apoptotic signal transduction systems where a variety of signal transduction systems participate at the upstream, appear to converge on caspase activating mechanisms at the downstream, the caspases being a series of cysteine protease *(Cell,* Vol. 91, p. 443, 1997), though their precise molecular mechanisms should be investigated in future.

Substances heretofore known as apoptosis inhibitors are, depending on species of the cells, a variety of growth factors and nutrient factors, physiological inhibitors such as hormones and the like, antioxidants such as N-acetyl-cysteine and the like, and modified peptide-type caspase inhibitors. Among them, some of peptide-type growth factors and neurotropic factors have been clinically used for the recovery of hematopoietic cells depleted after chemotherapy and for preventing cell death of neurons from neuro-degenerative diseases and trauma (*Proc. Natl. Acad. Sci. U.S.A.,* Vol. 90, p. 7951, 1993; *Nature,* Vol. 367, p. 368, 1994; *Proc. Natl. Acad. Sci. U.S.A.,* Vol. 89, p. 11249, 1992). The antioxidants and caspase inhibitors are only used in experiments of the cell level. Thus, it has been desired to develop an apoptosis inhibitor which is more stable *in vivo,* orally active, and a non-peptide type as well as low in molecular weight. Furthermore, since it is rare case that all apoptosis-triggering physiological factors and its inhibiting factors of the individual cells have been successfully identified in actual diseases, there is a demand for an entirely new type of cell death inhibitor which is also expected to be beneficial for the diseases where the factors are unidentified.

At present, Euro-Collins'solution and University of Wisconsin solution are generally used as organ preservation solutions for transplantation (*Ishoku (Transplantation),* Vol. 27, p. 172, 1992). Supplementation of antioxidants and radical scavengers to such preservation solutions in order to ameliorate damages of reactive oxygen has been reported to have beneficial effects on organ preservation (for example, *Ishoku (Transplantation),* Vol. 27, p. 15, 1992; Vol. 26, p. 62, 1991; Vol. 25, p. 596, 1990; *Trans Proc.,* Vol. 17, p. 1454, 1985). However, the organ preservation is not fully sufficient, and higher graft survival rate is still desired.

On the other hand, indolylmaleimide derivatives have been reported that they can inhibit protein kinases, especially protein kinase C (*J. Med. Chem.,* Vol. 35, p.177, 1992; *Bioorg. Med. Chem. Lett.,* Vol. 4, p. 2845, 1994), and their function as an anti-cancer drug is known. But, there is no report on their function of inhibiting cell death.

### Disclosure of the Invention

An object of the invention is to provide a compound useful for inhibiting death of cells, the drug being expected as a preventive or a remedy for the progress of various diseases wherein cell death participates in progress and exacerbation thereof.

As a result of the extensive studies for achieving the above, the present inventors have found that the below-mentioned indolylmaleimide derivatives exhibit a cell death inhibiting action and have accomplished the invention.

Namely, the invention provides a cell death inhibitor comprising, as an active ingredient, an indolylmaleimide derivative represented by the following formula (I): 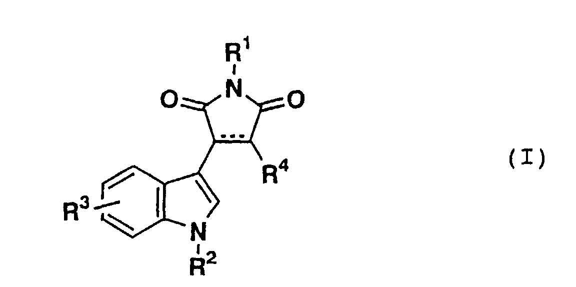 wherein R¹ represents an alkyl group which may be substituted, an alkenyl group which may be substituted, an aryl group which may be substituted, hydroxyl group, an alkoxyl group which may be substituted, an aryloxy group which may be substituted, an amino group which may be substituted, or hydrogen atom; R² represents hydrogen atom, an alkyl group which may be substituted, an alkenyl group which may be substituted, an alkynyl group which may be substituted, an aryl group which may be substituted, an acyl group which may be substituted, an alkoxy- or aryloxycarbonyl group which may be substituted, an alkyl- or arylthiocarbonyl group which may be substituted, an aminocarbonyl group which may be substituted, an alkyl- or arylsulfonyl group which may be substituted, an alkoxyl group which may be substituted, an aryloxy group which may be substituted, or hydroxyl group; R³ represents substituent(s) on an indole ring, and represents, number and position (2-, 4-, 5-, 6-, or 7-position as position number of the indole ring) of the substituent(s) and kinds of the substituent(s) may be the same or different, hydrogen atom, an alkyl group which may be substituted, an alkenyl group which may be substituted, an alkynyl group which may be substituted, an aryl group which may be substituted, an acyl group which may be substituted, an alkoxy- or aryloxycarbonyl group which may be substituted, an alkoxy- or aryloxycarbonyloxy group which may be substituted, an alkyl- or arylthiocarbonyl group which may be substituted, an aminocarbonyl group which may be substituted, an alkyl- or arylsulfonyl group which may be substituted, an alkoxyl group which may be substituted, an aryloxy group which may be substituted, an alkyl- or arylthio group which may be substituted, hydroxyl group, carboxyl group, cyano group, nitro group, an amino group which may be substituted, or a halogen atom; R⁴ represents hydrogen atom, an alkyl group which may be substituted, an alkenyl group which may be substituted, an alkynyl group which may be substituted, an aryl group which may be substituted (except 3-indolyl group), an acyl group which may be substituted, an alkoxy- or aryloxycarbonyl group which may be substituted, an alkyl- or arylthiocarbonyl group which may be substituted, an aminocarbonyl group which may be substituted, an alkyl- or arylsulfonyl group which may be substituted, an alkoxyl group which may be substituted, an aryloxy group which may be substituted, alkyl- or arylthio group which may be substituted, hydroxyl group, carboxyl group, cyano group, nitro group, or amino group which may be substituted; R² and R³, R² and R⁴, R³ and R⁴ may be combined to form a hydrocarbon chain which may be substituted; and in the formula, the bond accompanying a dotted line represents a double bond or a single bond; or a pharmaceutically acceptable salt thereof; a drug for treating or preventing progress of symptoms, through inhibiting death of neurons, of neurodegenerative diseases such as Alzheimer's disease, spinal muscular atrophy (SMA), amyotrophic lateral screrosis (ALS), Parkinson's disease, Huntington's disease, pigmentary degeneration of the retina, glaucoma, or cerebellar degeneration; a drug for treating or preventing progress of symptoms, through inhibiting death of neurons, of neonatal jaundice; a drug for treating or preventing progress of symptoms, through inhibiting death of cells, of myasthenia gravis; a drug for treating or preventing progress of symptoms, through inhibiting death of neurons, of brain ischemia from apoplexy and the like, and successive delayed neuronal death (DND); a drug for treating or preventing progress of symptoms, through inhibiting death of myocardial cells, of ischemic heart disease due to myocardial infarction, viral myocarditis, autoimmune myocarditis, myocardial disorders or death due to hypertrophic heart and heart failure, or arrythmogenic right ventricular cardiomyopathy; a drug for treating or preventing progress of symptoms, through inhibiting death of hepatic cells, of alcoholic hepatitis or viral hepatitis; a drug for treating or preventing progress of symptoms, through inhibiting death of renal cells, of renal diseases such as glomerulonephritis, hemolytic uremic syndrome and the like; a drug for treating or preventing progress of symptoms, through inhibiting excessive death of T-cells, of acquired immunodeficiency syndrome (AIDS); a drug for treating or preventing progress of symptoms, through inhibiting cell death, of inflammatory skin disorders such as toxic epidermal necrolysis (TEN), multiform exudative erythema and the like, alopecia, or graft versus host disease (GVH); a drug for treating or preventing disorders or side effects, through inhibiting cell death, of radiation disorders or disorders due to toxic agents including side effects due to drugs such as anti-cancer drugs, anti-viral drugs and the like; a drug for treating or preventing progress of symptoms, through inhibiting cell death, of sepsis; a drug for treating or preventing progress of symptoms, through inhibiting death of cells derived from bone marrow, of osteomyelo-dysplasia such as aplastic anemia and the like; a drug for treating or preventing progress of symptoms, through inhibiting cell death, of insulin dependent diabetes; a drug for treating or preventing progress of symptoms, through inhibiting death of neurons, of prion diseases; a drug for treating or preventing functional deficiency of transplanted organs, tissues or cells at transplantation of organs, tissues or cells; a preservative for organs, tissues and cells.

The following will explain the invention in detail.

### Best Mode for Carrying Out the Invention

The indolylmaleimide derivatives according to the invention can be synthesized according to known methods (for example, *J. Med. Chem.,* Vol. 35, p. 177, 1992; *Bioorg. Med. Chem. Lett.,* Vol. 4, p. 2845, 1994; *Bioorg. Med. Chem. Lett.,* Vol. 8, p. 47, 1998; *Tetrahedron Lett.,* Vol. 40, p. 1109, 1999; *SYNTHESIS, 443,* 1997; *Tetrahedron,* Vol. 55, p. 2363, 1999) or analogous methods thereof.

In the present description, the alkyl group in the "an alkyl group which may be substituted" may be any of linear, branched, or cyclic one, and may be exemplified by an alkyl group having 1 to 30 carbon atoms, more concretely, methyl group, ethyl group, propyl group, isopropyl group, butyl group, isobutyl group, s-butyl group, t-butyl group, pentyl group, isopentyl group, neopentyl group, hexyl group, heptyl group, octyl group, nonyl group, decyl group, cyclopropyl group, cyclobutyl group, cyclopentyl group, cyclohexyl group, cycloheptyl group, undecyl group, dodecyl group, tridecyl group, tetradecyl group, pentadecyl group, hexadecyl group, 14-methylpentadecyl group, 6-methylpentadecyl group, octadecyl group, eicosyl group, tetracosyl group, and the like.

In the present description, the alkenyl group in the "an alkenyl group which may be substituted" may be any of linear, branched, or cyclic one, and may be exemplified by an alkenyl group having 2 to 30 carbon atoms. Concrete examples thereof include allyl group, vinyl group, crotyl group, 1-penten-1-yl group, 2-penten-1-yl group, 3-penten-1-yl group, 1-hexen-1-yl group, 2-hexen-1-yl group, 3-hexen-1-yl group, 2-cyclohexenyl group, 2-cyclopentenyl group, 8-heptadecen-1-yl group, 8,11-heptadecadien-1-yl group, 8,11,14-heptadecatrien-1-yl group, 4,7,10,13-nonadecatetraen-1-yl group, 9-octadecen-1-yl group, 9,12-octadecadien-1-yl group, 9,12,15-octadecatrien-1-yl group, 6,9,12-octadecatrien-1-yl group, 5,8,11,14-eicosatetraen-1-yl group, 5,8,11,14,17-eicosapentaen-1-yl group, and 4,7,10,13,16,19-docosahexane-1-yl group.

In the present description, the alkynyl group in the "an alkynyl group which may be substituted" may be any of linear, branched, or cyclic one, and may be exemplified by an alkynyl group having 2 to 30 carbon atoms. Concrete examples thereof include ethynyl group, propargyl group, 1-pentyn-1-yl group, 2-pentyn-1-yl group, 3-pentyn-1-yl group, 1-octyn-1-yl group, and 8-heptadecyn-1-yl group.

In the present description, the aryl group in the "an aryl group which may be substituted" includes a heteroaryl group, and may be exemplified by phenyl group, naphthyl group, anthranyl group, pyrenyl group, biphenyl group, 4-pyridyl group, 2-pyridyl group, pyrimidinyl group, pyrazinyl group, piperazinyl group, pyrazolyl group, imidazolyl group, quinolyl group, pyrrolyl group, indolyl group, benzofuryl group, benzothiofuryl group, thiofuryl group, furyl group and the like. R⁴ is not a 3-indolyl group which may be substituted.

In the present description, the acyl group in the "an acyl group which may be substituted" or "an acyloxy group which may be substituted" may be any of linear, branched, cyclic, saturated, unsaturated, aliphatic or aromatic one, and may be exemplified by an acyl group having 2 to 30 carbon atoms, more concretely, acetyl group, propionyl group, isopropionyl group, pivaloyl group, oleoyl group, cyclohexylcarbonyl group, acryloyl group, crotonoyl group, benzoyl group, naphthoyl group, nicotinoyl group, and the like.

In the present description, the alkoxy- or aryloxycarbonyl group in the "an alkoxy- or aryloxycarbonyl group which may be substituted" may be any of linear, branched, cyclic, saturated, unsaturated, aliphatic or aromatic one. The examples include methoxycarbonyl group, ethoxycarbonyl group, propyloxycarbonyl group, isopropyloxycarbonyl group, butoxycarbonyl group, s-butoxycarbonyl group, t-butoxycarbonyl group, cyclopentyloxycarbonyl group, cyclohexyloxycarbonyl group, benzyloxycarbonyl group, allyloxycarbonyl group, phenyloxycarbonyl group, pyridyloxycarbonyl group, and the like.

In the present description, the alkyl- or arylthiocarbonyl group in the "an alkyl- or arylthiocarbonyl which may be substituted" may be any of linear, branched, cyclic, saturated, unsaturated, aliphatic or aromatic one. The examples include methylthiocarbonyl group, ethylthiocarbonyl group, propylthiocarbonyl group, isopropylthiocarbonyl group, butylthiocarbonyl group, t-butylthiocarbonyl group, cyclopentylthiocarbonyl group, cyclohexylthiocarbonyl group, benzylthiocarbonyl group, phenylthiocarbonyl group, pyridylthiocarbonyl group, and the like.

In the present description, the aminocarbonyl group in the "an aminocarbonyl which may be substituted" may be an unsubstituted carbamoyl group, or an carbamoyl group which is substituted by alkyl group(s) which may be substituted, aromatic group(s) which may be substituted, hydroxyl group, alkoxyl group(s) which may be substituted, amino group(s) which may be substituted, and the like. The examples include carbamoyl group, ethylaminocarbonyl group, propylaminocarbonyl group, isopropylaminocarbonyl group, butylaminocarbonyl group, t-butylaminocarbonyl group, cyclopentylaminocarbonyl group, cyclohexylaminocarbonyl group, benzylaminocarbonyl group, phenylaminocarbonyl group, pyridylaminocarbonyl group, benzyloxyaminocarbonyl group, and the like.

In the present description, the alkyl- or arylsulfonyl group in the "an alkyl or arylsulfonyl which may be substituted" may be any of linear, branched, cyclic, saturated, unsaturated, aliphatic or aromatic one. The examples include methanesulfonyl group, ethanesulfonyl group, benzenesulfonyl group, cyclohexanesulfonyl group, naphthalenesulfonyl group, and the like.

In the present description, the alkoxyl group or aryloxy group in the "an alkoxyl group or an aryloxy group which may be substituted" may be any of linear, branched, cyclic, saturated, unsaturated, aliphatic or aromatic one, and may be exemplified by an alkoxy group or an aryloxy group having 2 to 30 carbon atoms. The particular examples include methoxy group, ethoxy group, propyloxy group, t-butoxy group, allyloxy group, cyclopentyloxy group, cyclohexyloxy group, benzyloxy group, phenoxy group, and the like.

In the present description, the alkyl- or arylthio group in the "an alkyl- or arylthio which may be substituted" may be any of linear, branched, cyclic, saturated, unsaturated, aliphatic or aromatic one, and may be exemplified by an alkyl- or arylthio group having 2 to 30 carbon atoms. The particular examples include methylthio group, ethylthio group, propylthio group, t-butylthio group, allylthio group, cyclopentylthio group, cyclohexylthio group, benzylthio group, phenylthio group, and the like.

In the present description, the "an amino group which may be substituted" may be an unsubstituted amino group, or an amino group which is substituted by alkyl group(s), aromatic group(s), and the like. The examples include ethylamino group, propylamino group, isopropylamino group, butylamino group, t-butylamino group, benzylamino group, phenylamino group, pyridylamino group, piperazinyl group, indolinyl group, and the like.

In the present description, "a halogen atom" may be fluorine atom, chlorine atom, bromine atom, and iodine atom.

The examples of substituents which may be present in the above-mentioned alkyl group, alkenyl group, alkynyl group, aryl group, acyl group, alkoxy- or aryloxycarbonyl group, alkylthio- or arylthiocarboyl group, aminocarbonyl group, alkoxyl group or aryloxy group, alkyl- or arylthio group, amino group, and the like include alkyl groups, alkenyl groups, alkynyl groups, aryl groups, acyl groups, alkoxy- or aryloxycarbonyl group, alkylthio- or arylthiocarbonyl group, aminocarbonyl group, alkoxyl group, aryloxy group, alkyl- or arylthio group, and particular examples thereof are the same as mentioned above. The other substituents may be exemplified by halogen groups, nitro group, amino groups (which may be substituted by substituent(s) such as acyl group(s), alkoxy- or aryloxycarbonyl group(s), carbamoyl group(s), substituted sulfonyl group(s), alkyl group(s), cycloalkyl group(s), aryl group(s), and the like), cyano group, hydroxyl group, carboxyl group, and the like, as well as aralkyl groups such as benzyl group, phenethyl group, naphthylmethyl group, and the like.

As to the pharmaceutically acceptable salts, the compound having an acid part may formed a salt with an inorganic base or organic base, for example, an alkaline metal salt such as sodium salt, potassium salt, or the like; an alkaline earth metal salt such as calcium salt, magnesium salt, or the like; ammonium salt; an aliphatic or heteroaromatic amine salt such as triethylamine salt, ethanolamine salt, lysine salt, arginine salt, quinoline salt, or the like; a quaternary ammonium salt such as tetramethylammonium or the like. The compound having a basic part may form a salt with an inorganic or organic acid, for example, hydrochloride, bromate, iodate, sulfate, nitrate, phosphate, citrate, tartrate, malate, lactate, salicylate, malonate, fumarate, succiniate, oxalate, ascorbate, or the like.

The compound according to the invention may be applied as medicament in any form selected from various forms, for example, formulations for oral administration such as tablets, capsules, powders, granules, or liquids; and formulations for parenteral administration such as injections, rectal suppositories, formulations for external use on skin, inhalant, and the like.

Solid formulations can be prepared in a form of tablets, capsules, granules, or powders by themselves, or can be prepared with using suitable additive(s). Examples of such additives include sugars such as lactose or glucose; starches; fatty acids such as stearic acid; inorganic salts such as magnesium metasilicate aluminate or anhydrous calcium phosphate; synthetic polymers such as polyvinylpyrrolidone or polyalkylene glycol; fatty acid salts such as calcium stearate or magnesium stearate; alcohols such as stearyl alcohol or benzyl alcohol; synthetic cellulose derivatives such as methyl cellulose, ethyl cellulose, carboxymethyl cellulose, or hydroxypropyl cellulose; other conventional additives such as water, gelatin, talc, vegetable oils, acacia, or the like.

Liquid formulations are prepared in a form of suspensions, syrups, or injections by using suitable additive(s) conventionally used in liquid formulations such as water, alcohols, vegetable-derived oils including soybean oil, peanut oil, sesame oil, etc.

Especially, examples of suitable solvents for injections include distilled water for injection, aqueous lidocain hydrochloride solution, physiological saline, aqueous glucose solution, ethanol, liquids for intravenous injection such as aqueous solutions of citric acid and sodium citrate, electrolyte solution, and the like, or mixtures thereof. These injections may be a form of predissolved one, and also a form for dissolving before use, which is composed of powder itself or powder with suitable additive(s).

The rectal suppositories may be prepared either by melting an active ingredient and base material(s) such as cacao butter, tri-, di- and monoglyceride of a fatty acid, polyethylene glycol, and the like under heating; charging the melt into a mold; and then cooling it; or by dissolving an active ingredient into polyethylene glycol, soybean oil, or the like and then covering it with gelatin film.

In the preparation of formulations for external use on skin, an active ingredient is added to vaseline, bees wax, liquid paraffin, polyethylene glycol, etc., followed by either kneading it, if necessary under heating, to form ointments, or kneading it with an adhesive such as rosin, a polymer of alkyl acrylate, etc. and spreading the kneaded one on unwoven cloth such as polyethylene or the like to form tapes.

In the preparation of inhalant, an active ingredient is dissolved or dispersed into a propellant such as flon gas, and then a pressure container is filled up to form aerosols.

Preferred dosage of the compound according to the invention varies depending on kinds of the compositions blended, dosing times and diseases to be treated, and also ages, body weights, and symptoms of patients, but may be ordinarily in an amount of about 1-1000 mg a day, preferably 5 to 500 mg, and they may be administered in one or several dosage units per day.

The organs relating to the invention may be all organs, such as heart, lung, liver, kidney, pancreas, and intestine.

The tissues relating to the invention may be all tissues, such as skin, cornea, bone marrow, vascular systems, and bone.

In the invention, cells expected to have effects on maintenance of the cell function by transplantation or the preservation may be all types of cells (normal various cells, immortalized cell lines, cancer cells, and cells that are modified by genetic recombination techniques for disease treatment and research purposes), such as vascular cells, islet cells of Langerhans, epidermal cells, neuronal cell, and embryonic stem cells.

As far as an administration method is concerned, when the chemical compounds according to the invention are used for the preservation of organs, tissues, and cells, various routes can be selected. For example, the present compound or a pharmaceutically acceptable salt thereof can be added to a culture medium or preservation solution containing appropriate salts and nutrients. In case of organ transplantation, it can be also administered intravenously or at perfusion to a donor prior to the organ isolation.

The invention will be explained in detail in the following sections by way of Examples but the invention is, needless to say, not limited to the Examples.

### Examples

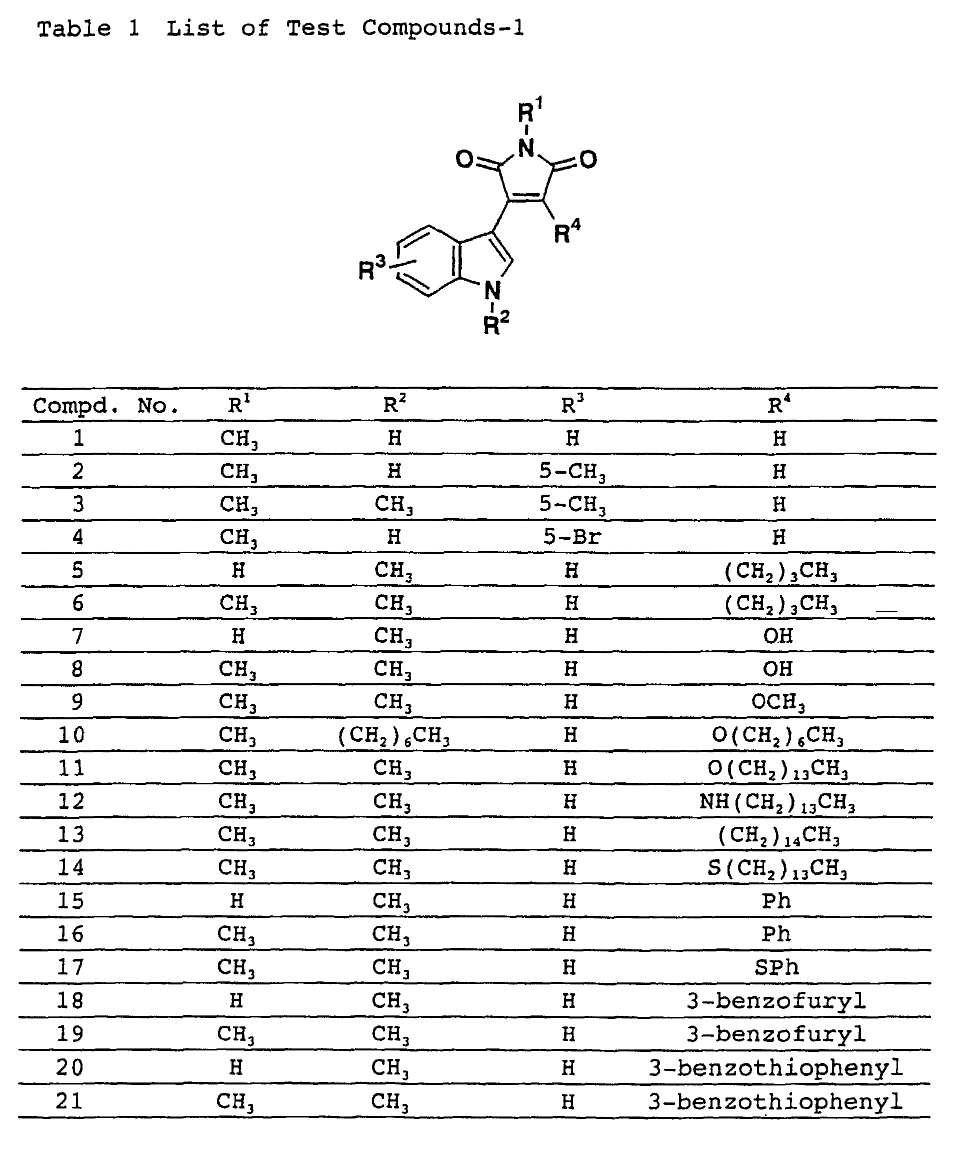

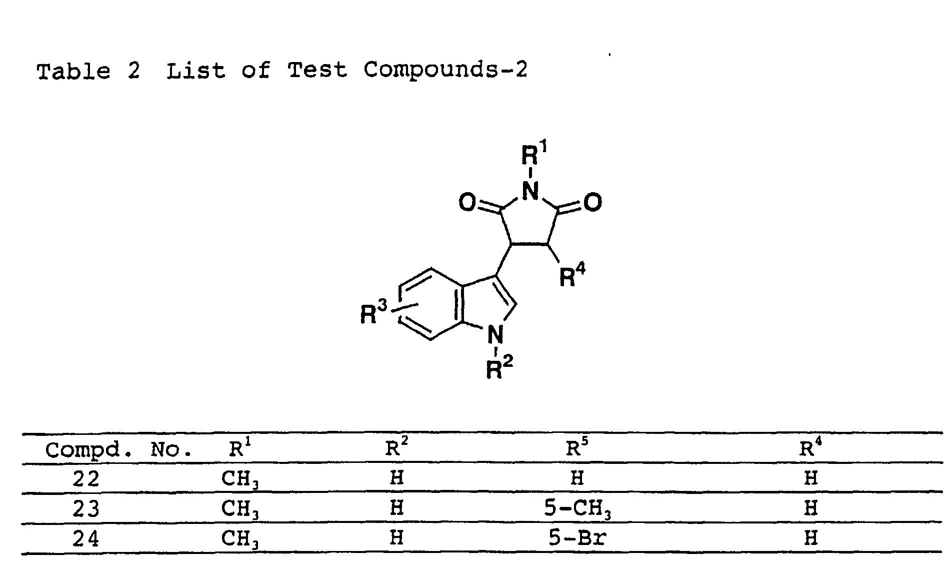

### Test Example 1

Porcine ovaries were washed with PBS buffer, and porcine ovarian granulosa cells (POGC) were collected by aspiration from follicles with a syringe. Cell fraction was recovered as a precipitate by centrifugation of the suspension. The operation of suspending the cell fraction into PBS buffer and subjecting the resulting suspension to centrifugation was repeated three times to wash the cells. The POGC obtained as a precipitate were suspended again into a culture medium (DMEM containing 10% fetal bovine serum) and cell clumps were disrupted by pipetting. The cell suspension was passed through a mesh to remove contaminating tissue fragments, and then pipetted in 24-well plates for cell culture. The cells were cultured for 2 to 3 days until the cells reached subconfluency (0.7 to 2×10⁵ cell/well) 2 days in a CO₂ incubator according to a conventional procedure (37°C, 5% CO₂). After the attaching cells were washed with serum-free medium, they were cultured in a serum-free DMEM (containing 5 µg/mL of transferrin, and 40 ng/mL of hydrocortisone, 4 mg/mL of bovine serum albumin (BSA), 100nM androstendione) and it was possible to continue to culture cells with relatively immature differentiation.

Addition of SNP (sodium nitroprusside, Na₂[Fe(CN)₅NO], 0.5 mM) known as an NO generating reagent to POGC cultured by the above method resulted in death of all the cells which was found on an observation after 6 hours under a light microscopy and an electron microscopy. The cell death was also confirmed by MTT assay and Trypan blue exclusion assay. Then, various concentrations of the test compounds were added to the present culture system and the cells were observed after 18 hours. The results are shown in Table 2 where a minimum effective concentration of each compound for more than 95% of inhibition of cell death is described.

**Table 2**

| Inhibiting Effects on Apoptosis of Porcine Ovarian Granulosa Cells by SNP stimulation | | | |
|---|---|---|---|
| Compound | MEC (µM) | Compound | MEC (µM) |
| 1 | 10 | 13 | 10 |
| 2 | 3 | 14 | 60* |
| 3 | 1 | 15 | 10 |
| 4 | 3 | 16 | 10 |
| 5 | 10 | 17 | 10 |
| 6 | 3 | 18 | 10 |
| 7 | 3 | 19 | 10 |
| 8 | 1 | 20 | 30 |
| 9 | 20 | 21 | 10 |
| 10 | 0.7 | 22 | 20 |
| 11 | 0.3 | 23 | 20 |
| 12 | 0.3 | 24 | 20 |
| MEC : Minimum effective concentration | | | |

| | | | |
|---|---|---|---|
| * Partial inhibition (60% inhibition) | | | |

Compound 1 *(Tetrahedron,* Vol. 55, p. 2363, 1999), Compound 7 (*Bioorg. Med. Chem. Lett.,* Vol. 8, p. 47, 1998), and Compounds 22-24 *(SYNTHESIS, p.* 443, 1997) used in the test example were synthesized according to known methods. Synthesis of Compounds 2-6 and 8-21 were shown in the following referential examples.

### Referential Example 1

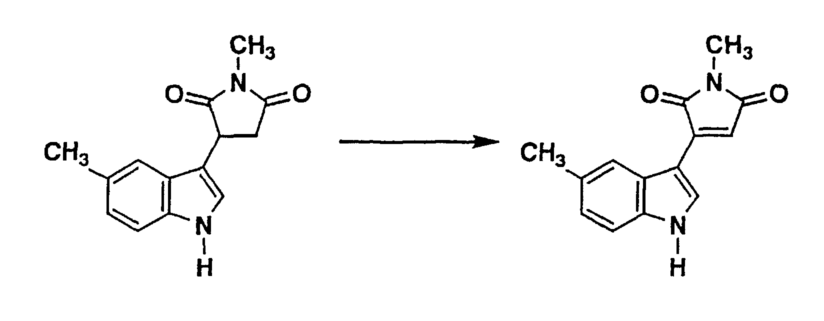

To a solution of Compound 23 (100 mg, 0.41 mmol) synthesized according to a known method (*SYNTHESIS,* p. 443, 1997) in 1,4-dioxane (5 mL) was added a solution of 2,3-dichloro-5,6-dicyano-1,4-benzoquinone (DDQ) (90 mg, 0.41 mmol) in 1,4-dioxane (5 mL) slowly, and the whole was stirred at room temperature for overnight. After filtration of the reaction mixture the filtrate was concentrated under reduced pressure. The residue was purified by column chromatography over silica gel (ethyl acetate:n-hexane=2:1) to obtain Compound 2 (45.7 mg, 46%) as yellow solid.
mp 225-230°C
¹H NMR(DMSO-d₆) δ 2. 44(s, 3H), 2. 94(s, 3H), 6. 88(s, 1H), 7. 08(d, J=8. 2Hz, 1H), 7. 40(d, J=8. 2Hz, 1H), 7. 79(s, 1H), 8. 34(d, J=2. 8Hz, 1H), 11. 91(brs, 1H). IR(KBr) 3200, 2920, 1745, 1690, 1610, 1440, 800, 625cm⁻¹.
MS m/z 240 (M⁺)

### Referential Example 2

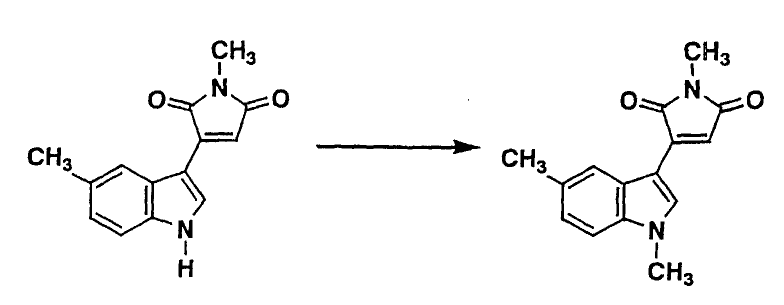

To a solution of Compound 2 (25 mg, 0.1 mmol) in DMF (1 mL) was added potassium carbonate (22 mg, 0.16 mmol) and methyl iodide (9.7 µL, 0.16 mmol), and the whole was stirred for 4 hours. To the reaction mixture was added saturated aqueous sodium chloride solution, and the whole mixture was extracted with ethyl acetate. The extract was dried over sodium sulfate, and then concentrated under reduced pressure. The residue was purified by column chromatography over silica gel (ethyl acetate:n-hexane=1:2) to obtain Compound 3 (26 mg, 98%) as yellow solid.
mp 249-251°C
¹H NMR(CDCl₃) δ 2. 51(s, 3H), 3. 07(s, 3H), 3. 84(s, 3H), 6. 57(s, 1H), 7. 17(d, J=8 . 3Hz, 1H), 7. 28(d, J=8. 3Hz, 1H), 7. 56(s, 1H), 8. 26(s, 1H).
IR(KBr) 3450, 1760, 1690, 1450, 1370, 1110, 800cm⁻¹
MS m/z 254(M⁺)

### Referential Example 3

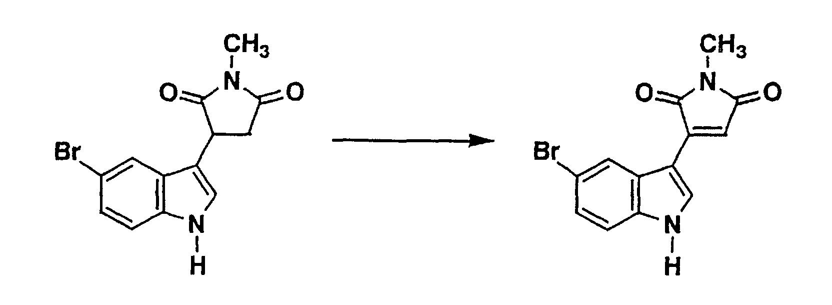

To a solution of Compound 24 (80 mg, 0.26 mmol) synthesized according to a known method (*SYNTHESIS,* p. 443, 1997) in 1,4-dioxane (4 mL) was added a solution of DDQ (60 mg, 0.26 mmol) in 1,4-dioxane (4 mL) slowly, and the whole was stirred at room temperature for overnight. After filtration of the reaction mixture the filtrate was concentrated under reduced pressure. The residue was purified by column chromatography over silica gel (ethyl acetate:n-hexane=1:1) to obtain Compound 4 (48 mg, 60%) as yellow solid.
mp 250-253°C
¹H NMR(DMSO-d₆) δ 2. 99(s, 3H), 7. 04(s, 1H), 7. 42(dd, J=1. 5, 8. 6Hz, 1H), 7. 55(d , J=8. 6Hz, 1H), 8. 20(d, J=1. 5Hz, 1H), 8. 45(d, J=2. 9Hz, 1H, 12. 26(brs, 1H).
IR(KBr) 3290, 1690, 1610, 1455, 1435, 1390, 1130, 1110, 810cm⁻¹.
MS m/z 304 (M⁺)

### Referential Example 4

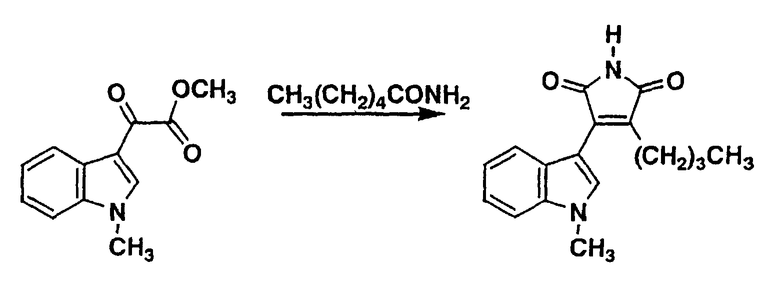

To a solution of methyl (1-methyl-indol-3-yl)glyoxylate (100 mg, 0.46 mmol) synthesized according to a known method (*J. Org. Chem.,* Vol. 63, p. 6053, 1998) and hexanamide (58 mg, 0.51 mmol) in DMF (1 mL) was added a solution of potassium tert-butoxide (110 mg, 1.01 mmol) in DMF (2 mL), and the whole was stirred at 45°C for 3 hours. After addition of 1N aqueous hydrochloric acid, whole was stirred at 45°C for 1 hour. After cooling to room temperature the mixture was extracted with ethyl acetate. The extract was washed with saturated aqueous sodium chloride solution, dried over sodium sulfate, and then concentrated under reduced pressure. The residue was purified by column chromatography over silica (ethyl acetate:n-hexane=2:1) to obtain Compound 5 (100 mg, 76.9%) as yellow solid.
mp 170-174°C
¹H NMR(CDCl₃) δ 0. 86(t, J=7. 4Hz, 3H), 1. 33(tt, J=7. 4, 7. 4Hz, 2H), 1. 56-1. 66(m , 2H), 2.66(t, J=7.4Hz, 2H), 3. 87(s, 3H), 7. 22(t, J=7. 8Hz, 1H), 7. 31(t, J=7. 8Hz, 1 H), 7. 38(d, J=7. 8Hz, 1H), 7. 51(s, 1H), 7. 61(brs, 1H), 7. 63(d, J=7. 8Hz, 1H).
IR(KBr) 3190, 3150, 2960, 1765, 1700, 1610, 1510, 1360, 1340, 1220, 740cm⁻¹.
MS m/z 282(M⁺)

### Referential Example 5

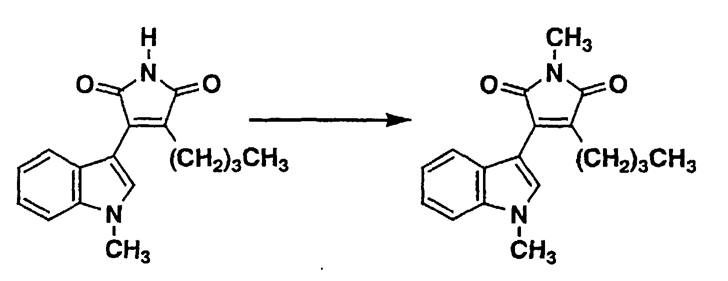

To a solution of Compound 5 (180 mg, 0.64 mmol) in DMF (5 mL) was added sodium hydride (60 to 72%, oily, 38.3 mg) at 0 °C, and the whole was stirred for 10 minutes. Methyl iodide (60 µL, 0.96 mmol) was added, and the mixture was gradually warming to room temperature and stirred for 2 hours. Saturated aqueous sodium chloride solution was added and the mixture was extracted with ethyl acetate. The extract was dried over sodium sulfate and concentrated under reduced pressure. The residue was purified by column chromatography over silica gel (ethyl acetate:n-hexane=1:1) to obtain Compound 6 (160 mg, 84%) as orange solid.
mp 96-99°C
¹H NMR(CDCl₃) δ 0. 86(t, J=7. 4Hz, 3H), 1. 32(tt, J=7. 4, 7. 4Hz, 2H), 1. 55-1. 64(m , 2H), 2. 67(t, J=7. 4Hz, 2H), 3. 09(s, 3H), 3. 86(s, 3H), 7. 22(t, J=7. 9Hz, 1H), 7. 30( t, J=7. 9Hz, 1H), 7. 37(d, J=7. 9Hz, 1H), 7. 49(s, 1H), 7. 63 (d, J=7. 9Hz, 1H).
IR(KBr) 3120, 2960, 2940, 1760, 1690, 1630, 1520, 1450, 1370, 740cm⁻¹.
MS m/z 296(M⁺)

### Referential Example 6

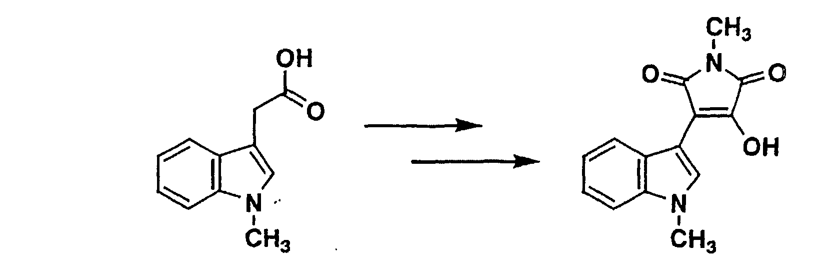

To a solution of 1-methyl-3-indolacetic acid (200 mg, 1.06 mmol) and N-hydroxysuccinimide (120 mg, 1.06 mmol) in THF (4 mL) was added a solution of DCC (220 mg, 1.06 mmol) in THF (4 mL), and the whole was stirred at room temperature for 1 day. After removal of insoluble material by filtration through celite pad, the filtrate was concentrated under reduced pressure. The residue was purified by column chromatography over silica gel (ethyl acetate:n-hexane=1:1) to obtain 1-methyl-3-indolacetic acid N-hydroxysuccinimide ester (270 mg, 89%) as colorless solid.
¹H NMR(CDCl₃) δ 2. 76(brs, 4H), 3. 75(s. 3H), 4. 07(s, 2H), 7. 11(s, 1H), 7. 12-7. 3 3(m, 3H), 7. 60(d, J=7. 7Hz, 1H).

To a solution of 1-methyl-3-indolacetic acid N-hydroxysuccinimide ester (100 mg, 0.35 mmol) in THF (3 mL) were added triethylamine (49 µL, 0.35 mmol) and methylamine hydrochloride (24 mg, 0.35 mmol), and the mixture was stirred at room temperature for 1 day. After removal of insoluble material by filtration through celite pad, the filtrate was concentrated under reduced pressure. The residue was purified by column chromatography over silica gel (chloroform:methanol=6:1) to obtain N-methyl-(1-methylindol)acetamide (70 mg, 100%) as colorless solid.
mp 94-97°C
¹H NMR(CDCl₃) δ 2. 70(d, J=4. 5Hz, 3H), 3. 72(s, 2H), 3. 79(s, 3H), 5. 67(brs, 1H), 6. 99 (s, 1H), 7. 15(t, J=7. 9Hz, 1H), 7.27(t, J=7. 9Hz, 1H), 7. 35(d, J=7.9Hz, 1H), 7. 59 (d, J=7. 9Hz, 1H).
IR(KBr) 3350, 3080, 2920, 1640, 1560, 1250, 1160, 740cm⁻¹.
MSm/z 202(M⁺)

To a solution of N-methyl-(1-methylindol)acetamide (526 mg, 2.6 mmol) and dimethyl oxalate (400 mg, 3.39 mmol) in DMF (10 mL) was added a solution of potassium tert-butoxide (730 mg, 6.51 mmol) in DMF (5 mL), and the whole was stirred at room temperature for 6 hours. The reaction mixture was added into water (50 mL), acidified with 1N aqueous hydrochloric acid after addition of ethyl acetate (50 mL), and extracted with ethyl acetate. The extract was washed with saturated aqueous sodium chloride solution, dried over sodium sulfate, and then concentrated under reduced pressure. The residue was purified by column chromatography over silica gel (ethyl acetate:n-hexane=2:1) to obtain Compound 8 (443 mg, 66%) as red solid.
mp 215-219°C
¹H NMR(DMSO-d₆) δ 2. 92(s, 3H), 3. 83(s, 3H), 7. 08(t, J=7. 8Hz, 1H), 7. 20(t, J=7. 8Hz, 1H), 7. 45(d, J=7. 8Hz, 1H), 7. 83 (s, 1H), 8. 12(d, J=7.8Hz, 1H), 11. 90 (brs. 1H).
IR(KBr) 3500, 3250, 1760, 1685, 1510, 1455, 1355, 1320, 1240, 750cm⁻¹.
MS m/z 256(M⁺)

### Referential Example 7

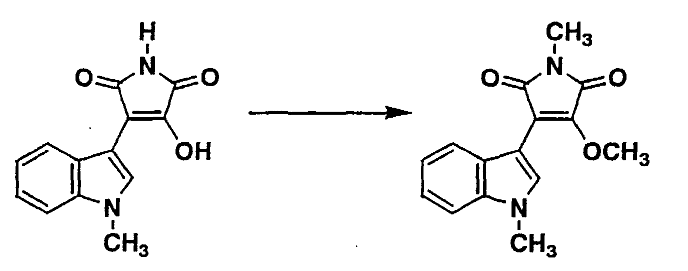

To a solution of Compound 7 (40 mg, 0.13 mmol) synthesized according to a known method (*Bioorg. Med. Chem. Lett.,* Vol. 8, p. 47, 1998) in DMF (5 mL) was added sodium hydride (60 to 72%, oily, 33 mg) at 0 °C, and the whole was stirred for 10 minutes. Methyl iodide (39 µL, 0.62 mmol) was added, and the mixture was gradually warming to room temperature and stirred for 3 hours. Saturated aqueous sodium chloride solution was added and the mixture was extracted with ethyl acetate. The extract was dried over sodium sulfate and concentrated under reduced pressure. The residue was purified by column chromatography over silica gel (dichloromethane:methanol=10:1) to obtain Compound 9 (18 mg, 16%) as orange solid.
mp 142-145°C
¹H NMR(CDCl₃) δ 3. 05(s, 3H), 3. 83(s, 3H), 4. 21(s, 3H), 7. 19(t, J=8. 0Hz, 1H), 7. 28 (t, J=8. 0Hz, 1H), 7. 33 (d, J=8. 0Hz, 1H), 7. 76 (s, 1H), 7. 89(d, J=8. 0Hz, 1H).
IR(KBr) 3450, 2950, 1760, 1700, 1650, 1520, 1445, 1380, 1300, 1250, 750cm⁻¹.
MS m/z 270(M⁺)

### Referential Example 8

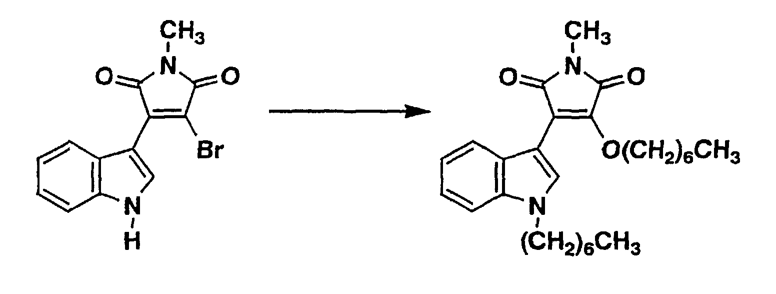

To a suspension of sodium hydride (60 to 72%, oily, 34 mg) in DMF (0.5 mL) was added a solution of 2-bromo-3-(1H-indol-3-yl)-N-methylmaleimide (130 mg, 0.425 mmol) synthesized according to a known method *(Tetrahedron,* Vol. 44, p. 2887, 1988) in DMF (2 mL) at room temperature, and the whole was stirred at room temperature for 30 minute. n-Heptyl bromide (0.60 mL, 4.3 mmol) was added, and the mixture was stirred at 40°C for 1.5 hours. After removal of DMF under reduced pressure and addition of water the reaction mixture was extracted with dichloromethan and ethyl acetate. Combined organic layers were washed with water, dried over sodium sulfate and concentrated under reduced pressure. The residue was purified by column chromatography over silica gel (ethyl acetate:n-hexane=1:10-1:1) to obtain 2-bromo-3-(1-n-heptyl-1H-indol-3-yl)N-methylmaleimide (39 mg, 23%) and Compound 10 (22 mg, 16%) as red solids.
Compound 10: mp 39-41°C
¹H NMR(CDCl₃) δ 0. 8-1. 05(m, 6H), 1. 07-1. 52(m, 16H), 1. 71-2. 00(m, 4H), 3. 05(s , 3H), 4. 14(t, J=7. 2Hz, 2H), 4. 54(t, J=6. 6Hz, 1H), 7. 16(t, J=7. 8Hz, 1H), 7. 25(t, J =7. 8Hz, 1H), 7. 35 (d, J=7. 8Hz, 1H), 7. 84 (s, 1H), 8. 07 (d, J=7. 8Hz, 1H).
IR(KBr) 3450, 3925, 2850, 1760, 1700, 1650, 1520, 1460, 1440, 1380, 740cm⁻¹.
MS m/z 438(M⁺)

### Referential Example 9

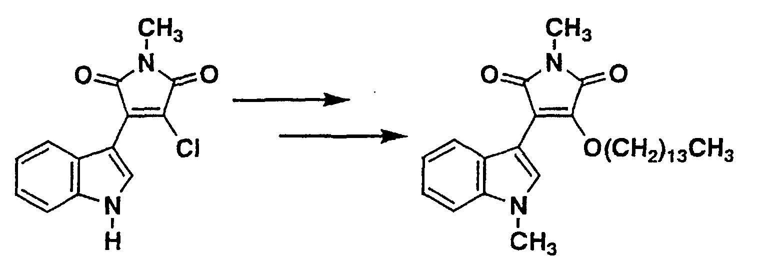

To a solution of 2-chloro-3-(1H-indol-3-yl)-N-methylmaleimide (579 mg, 2.2 mmol) synthesized according to a known method (*SYNTHESIS,* p. 1151, 1995) in DMF (10 mL) was added potassium carbonate (900 mg, 6.7 mmol) and methyl iodide (0.4 mL, 6.7 mmol), and the whole was stirred for 2 hours. To the reaction mixture was added saturated aqueous sodium chloride solution, and the whole mixture was extracted with ethyl acetate. The extract was dried over sodium sulfate, and then concentrated under reduced pressure. The residue was purified by column chromatography over silica gel (ethyl acetate:n-hexane=1:2) to obtain 2-chloro-3-(1-methyl-lH-indol-3-yl)-N-methylmaleimide (433 mg, 81%) as yellow solid.
mp 174-176°C
¹H NMR(CDCl₃) δ 3. 08(s, 3H), 3. 82(s, 3H), 7. 17-7. 36(m, 3H), 7. 85(s, 1H), 8. 01
(d, J=7. 6Hz, 1H).
IR(KBr) 1770, 1700, 1620, 1510, 1440, 1380, 1210, 1125, 750, 740cm⁻¹.
MS m/z 274(M⁺)

To a solution of decanol (78 mg, 0.36 mmol) in THF (0.5 mL) was added sodium hydride (60 to 72%, oily, 15 mg), and the whole was stirred for 10 minutes. To this mixture was added a solution of 2-chloro-3-(1-methyl-lH-indol-3-yl)-N-methylmaleimide (50 mg, 0.18 mmol) in THF (1 mL) at room temperature, and the mixture was stirred for 2 hours. Saturated aqueous ammonium chloride solution was added and the mixture was extracted with ethyl acetate. The extract was washed with saturated sodium chloride solution, dried over sodium sulfate, and concentrated under reduced pressure. The residue was purified by column chromatography over silica gel (ethyl acetate:n-hexane=1:3) to obtain Compound 11 (76 mg, 92%) as red solid.
mp 69-72 °C
¹H NMR(CDCl₃) δ 0. 88(t, J=7. 0Hz, 3H), 1. 15-1. 45 (m, 22H), 1. 76(tt, J=7.0, 7. 0 Hz, 2H), 3. 05(s, 3H), 3. 83(s, 3H), 4. 53(t, J=7. 0Hz, 2H), 7. 17(t, J=7. 6Hz, 1H), 7. 2 7(t, J=7. 6Hz, 1H), 7. 32(d, J=7. 6Hz, 1H), 7. 78(s, 1H), 8. 07(d, J=7. 6Hz, 1H).
IR(KBr) 2925, 2850, 1760, 1710 1700, 1520, 1440, 1380, 740cm⁻¹.
MS m/z 452(M⁺)

### Referential Example 10

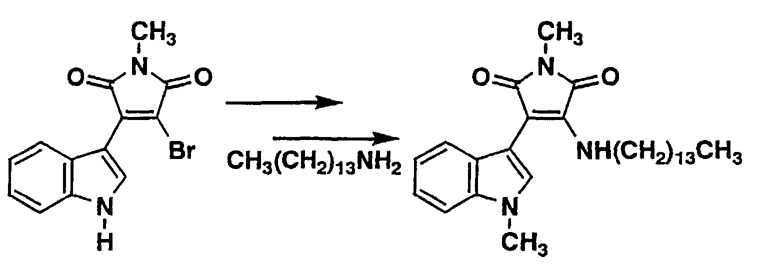

To a solution of 2-bromo-3-(1H-indol-3-yl)-N-methylmaleimide (100 mg, 0.33 mmol) synthesized according to a known method *(Tetrahedron,* Vol. 44, p. 2887, 1988) in DMF (5 mL) was added potassium carbonate (900 mg, 6.7 mmol) and methyl iodide (0.4 mL, 6.7 mmol) at 0°C, and the whole was stirred for 2 hours. After warming to room temperature saturated aqueous sodium chloride solution was added to the mixture, and the whole mixture was extracted with ethyl acetate. The extract was dried over sodium sulfate, and then concentrated under reduced pressure. The residue was purified by column chromatography over silica gel (ethyl acetate:n-hexane=1:1) to obtain 2-bromo-3-(1-methyl-1H-indol-3-yl)-N-methylmaleimide (99 mg, 95%) as red solid.
mp 155-158°C
¹H NMR(CDCl₃) δ 3.17(s, 3H), 3. 89(s, 3H), 7. 16-7.41 (m, 4H), 8.05-8.11 (m, 1H).
IR(KBr) 1760, 1700, 1585, 1510, 1430, 1375, 1230, 1150, 1120, 980, 800, 730cm⁻¹.
MS m/z 318(M⁺)

To a solution of 2-bromo-3-(1-methyl-1H-indol-3-yl)-N-methylmaleimide (50 mg, 0.16 mmol) in dichloromethane (2 mL) was added tetradecylamine (334 mg, 1.6 mmol), and the mixture was stirred at room temperature for 3 days. The reaction mixture was concentrated under reduced pressure, and the residue was purified by column chromatography over silica gel (ethyl acetate:n-hexane=1:2) to obtain Compound 12 (60 mg, 88%) as red solid.
mp 58-62°C
¹H NMR(CDCl₃) δ 0. 88(t, J=6. 8Hz, 3H), 0. 97-1. 40(m, 24H), 3. 06(s, 3H), 3. 13(dt , J=6. 8, 6. 8Hz, 2H), 3. 82 (s, 3H), 5. 15-5. 21 (m, 1H), 7.11(s, 1H), 7. 12(t, J=8. 0Hz, 1H), 7. 22(t, J=8. 0Hz, 1H), 7. 31(d, J=8. 0Hz, 1H), 7. 48(d, 1=8.0Hz, 1H).
IR(KBr) 3350, 2940, 1860, 1760, 1660, 1545, 1460, 740cm⁻¹.
MS m/z 451(M⁺)

### Referential Example 11

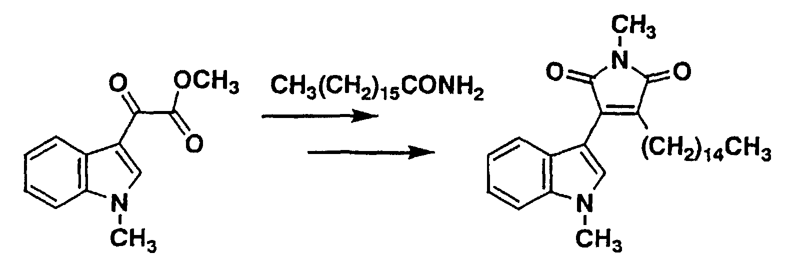

To a solution of methyl (1-methyl-indol-3-yl)glyoxylate (100 mg, 0.46 mmol) synthesized according to a known method (*J*. *Org. Chem.,* 63, 6053, 1998) and n-heptadecanamide (130 mg, 0.51 mmol) in DMF (1 mL) was added a solution of potassium tert-butoxide (110 mg, 1.01 mmol) in DMF (2 mL), and the whole was stirred at 45°C for 3 hours. After addition of 1N aqueous hydrochloric acid, whole was stirred at 45°C for 1.5 hour. The mixture was cooled to room temperature, and extracted with ethyl acetate. The extract was washed with saturated aqueous sodium chloride solution, dried over sodium sulfate, and then concentrated under reduced pressure. The residue was purified by column chromatography over silica (chloroform:methanol=50:1) to obtain 2-pentadecyl-3-(1-methyl-1H-indol-3-yl)maleimide (122 mg, 61%) as yellow solid.
mp 143-145°C
¹H NMR(CDCl₃) δ 0. 88(t, J=6. 8Hz, 3H), 1. 15-1. 35(m, 24H), 1. 56-1. 66(m, 2H), 2. 65(t, J=7. 8Hz, 2H), 3. 87(s, 3H), 7. 22(t, J=8. 0Hz, 1H), 7. 31(t, J=8. 0Hz, 1H), 7.35 (brs, 1H), 7. 37(d, J=8. 0Hz, 1H), 7. 50(s, 1H), 7. 62(d, J=8. 0Hz, 1H).
IR(KBr) 3190, 2925, 2850, 1770, 1700, 1620, 1520m 1470, 1360, 1340, 740cm⁻¹.
MS m/z 436(M⁺)

To a solution of 2-pentadecyl-3-(1-methyl-1H-indol-3-yl)maleimide (50 mg, 0.11 mmol) in DMF (1 mL) was added sodium hydride (60 to 72%, oily, 6.9 mg) and methyl iodide (11 µL, 0.17 mmol), and the whole was stirred for 1 hour. Saturated aqueous sodium chloride solution was added and the mixture was extracted with ethyl acetate. The extract was dried over sodium sulfate and concentrated under reduced pressure. The residue was purified by column chromatography over silica gel (ethyl acetate:n-hexane=2:1) to obtain Compound 13 (44.5 mg, 90%) as yellow solid.
mp 92-94°C
¹H NMR(CDCl₃) δ 0. 88(t, J=6. 8Hz, 3H), 1. 14-1. 34 (m, 24H), 1. 52-1. 64 (m, 2H), 2. 66(t, J=7. 8Hz, 2H), 3. 08(s, 3H), 3. 87(s, 3H), 7. 21(t, J=8. 0Hz, 1H), 7. 31(t, J=8.0 Hz, 1H), 7. 37(d, J=8. 0Hz, 1H), 7. 49(s, 1H), 7. 62(d, J=8. 0Hz, 1H).
IR(KBr) 2925, 2850, 1760, 1710, 1690, 1635, 1520, 1450, 1375, 1240, 740cm⁻¹.
MS m/z 450(M⁺)

### Referential Example 12

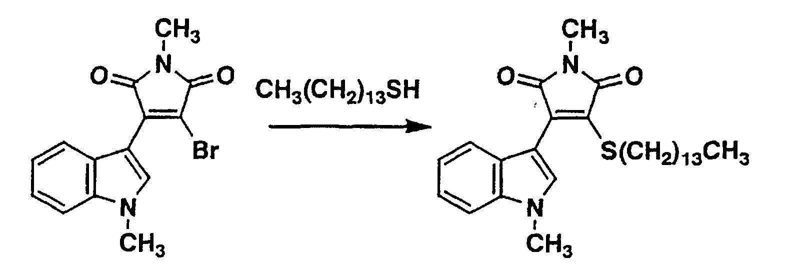

To a solution of 2-bromo-3-(1-methyl-1H-indol-3-yl)-N-methylmaleimide (30 mg, 0.094 mmol) synthesized according to the referential example 10 in THF (0.5 mL) were added N,N-diisopropylethylamine (5.3 µL, 0.037 mmol) and n-tetradecylmercaptane (51 µL, 0.19 mmol), and the mixture was stirred at room temperature for 2 days. The reaction mixture was concentrated under reduced pressure, and the residue was purified by column chromatography over silica gel (ethyl acetate:n-hexane=3:1) to obtain Compound 14 (35 mg, 79%) as red solid.
mp 61-64°C
¹H NMR(CDCl₃) δ 0. 88(t, J=7. 5Hz, 3H), 1. 14-1. 35(m, 22H), 1. 56(tt, J=7. 5, 7. 5Hz, 2H), 3. 10(s, 3H), 3. 11(t, J=7. 5Hz, 2H), 3. 86(s, 3H), 7. 23(t, J=8. 0Hz, 1H), 7. 30(t , J=8. 0Hz, 1H), 7. 36(d, J=8. 0Hz, 1H), 7. 64(s, 1H), 8. 02(d, J=8. 0Hz, 1H).
IR(KBr) 3450, 2925, 2850, 1760, 1690, 1575, 1440, 1375, 1120, 740cm⁻¹.
MS m/z 468(M⁺)

### Referential Example 13

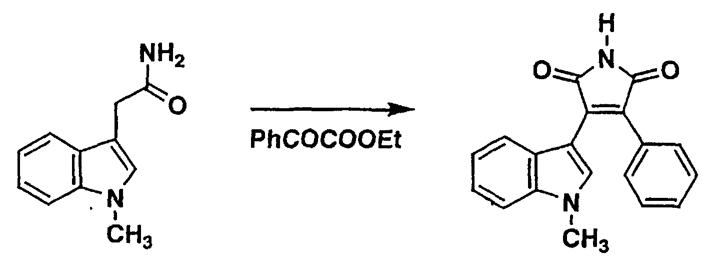

To a solution of 1-methyl-indol-3-acetamide (50 mg, 0.27 mmol) synthesized according to a known method (*J*. *Org. Chem.,* 63, 6053, 1998) and ethyl phenylglyoxylate (52 mg, 0.29 mmol) in DMF (1 mL) was added a solution of potassium tert-butoxide (66 mg, 0.59 mmol) in DMF (1.5 mL), and the whole was stirred at 45°C for 4 hours. Reaction mixture was poured into water, and ethyl acetate (10 mL) was added. After acidification with 1N aqueous hydrochloric acid, the mixture was extracted with ethyl acetate. The extract was washed with saturated aqueous sodium chloride solution, dried over sodium sulfate, and then concentrated under reduced pressure. The residue was purified by column chromatography over silica gel (ethyl acetate:n-hexane=5:1) to obtain Compound 15 (71 mg, 88%) as yellow solid.
mp 255-257°C
¹H NMR(DMSO-d₆) δ 3. 89(s, 3H), 6. 29(d, J=8. 0Hz, 1H), 6. 70( t, J=8. 0Hz, 1H), 7. 1 1 (t, J=8. 0Hz, 1H), 7. 29-7. 37(m, 3H), 7. 39(dd, J=8. 0, 1. 7Hz, 2H), 7. 47(d, 1=8.0Hz 1H),8.03(s, 1H), 11.0 5(brs, 1H).
IR(KBr) 3156, 3050, 1760, 1700, 1620, 1515, 1335, 1250cm⁻¹.
MS m/z 302(M⁺)

### Referential Example 14

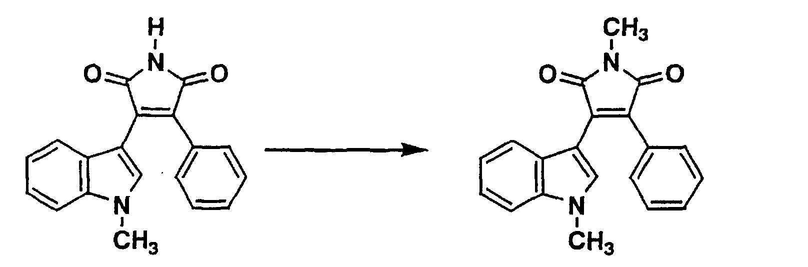

To a solution of Compound 15 (50 mg, 0.16 mmol) in DMF (1 mL) was added sodium hydride (60 to 72%, oily, 9.9 mg) at 0 °C, and the whole was stirred for 10 minutes. Methyl iodide (15 µL, 0.25 mmol) was added, and the mixture was gradually warming to room temperature and stirred for 2 hours. Saturated aqueous sodium chloride solution was added and the mixture was extracted with ethyl acetate. The extract was dried over sodium sulfate and concentrated under reduced pressure. The residue was purified by column chromatography over silica gel (ethyl acetate:n-hexane=1:1) to obtain Compound 16 (49 mg, 93%) as orange solid.
mp 242-246°C
¹H NMR (CDCl₃) δ 3. 16(s, 3H) , 3. 88(s, 3H) , 6. 40(d, J=8. 0Hz, 1H), 6. 78(t , J=8. 0Hz, 1H), 7. 16(t. J=8. 0Hz, 1H), 7. 27-7. 36(m, 4H), 7. 51(d, J=8. 0Hz, 2H), 7. 94(s, 1H).
IR(KBr) 3150, 3050, 1760, 1695, 1625, 1520, 1375, 1250cm⁻¹.
MS m/z 316 (M⁺)

### Referential Example 15

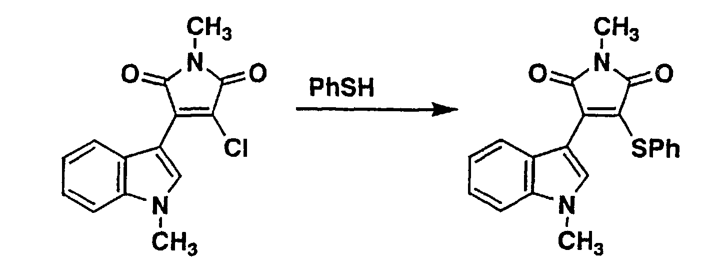

To a solution of 2-chloro-3-(1-methyl-1H-indol-3-yl)-N-methylmaleimide (75 mg, 0.27 mmol) in THF (1 mL) were added N,N-diisopropylethylamine (15 µL, 0.19 mmol) and thiophenol (28 µL, 0.19 mmol), and the mixture was stirred at room temperature for 2 days. The reaction mixture was concentrated under reduced pressure, and the residue was purified by column chromatography over silica gel (ethyl acetate:n-hexane=3:1) to obtain Compound 17 (69 mg, 72%) as red solid.
mp 142-144°C
¹H NMR(CDCl₃) δ 3. 09(s, 3H), 3. 83(s, 3H), 7. 13-7. 24(m, 4H), 7. 26-7. 36(m, 4H), 7. 75(s, 1H), 8. 07(d, J=8. 1Hz, 1H).
IR(KBr) 1765, 1700, 1590, 1440, 1370, 1230, 1120, 740cm⁻¹.
MS m/z 348(M⁺)

### Referential Example 16

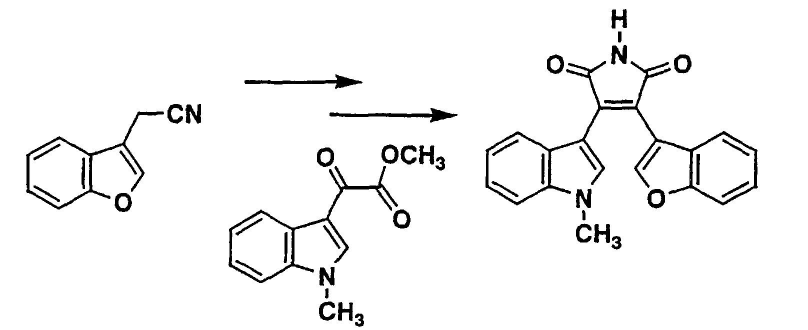

To a solution of benzofuran-3-acetonitrile (120 mg, 0.76 mmol) synthesized according to a known method (*J*. *Med. Chem.,* Vol. 35, p. 1176, 1992) in tert-butyl alcohol (2 mL) was added potassium hydride (340 mg, 6.08 mmol), and the whole was refluxed for 1.5 hours. After cooling to room temperature and acidification with 1N hydrochloric acid, resulting solid was filtrated, washed with water and diethyl ether gave benzofuran-3-acetamide (100 mg, 75%) as brown solid.
¹H NMR(DMSO-d₆) δ 3. 48(s, 2H), 6. 98(brs, 1H), 7. 25(t, J=7. 4Hz, 1H), 7. 31(t, J= 7. 4Hz, 1H), 7. 53(brs, 1H), 7. 54 (d, J=7. 4Hz, 1H), 7.63(d, J=7. 4Hz, 1H), 7. 81(s, 1H).
IR(KBr) 3355, 3200, 1665, 1640, 1460, 1420, 1410, 1100, 755cm⁻¹.
MS m/z 175(M⁺)

To a solution of benzofuran-3-acetamide (50 mg, 0.31 mmol) and methyl (1-methylindol-3-yl)glyoxylate (78 mg, 0.35 mmol) synthesized according to a known method (*J. Org.* *Chem.,* Vol. 63, p. 6053, 1998) in DMF (1 mL) was added a solution of potassium tert-butoxide (78 mg, 0.69 mmol) in DMF (1.5 mL), and the whole was stirred at 45°C for 6 hours. After cooling to room temperature and addition of 1N aqueous hydrochloric acid, the mixture was extracted with ethyl acetate. The extract was washed with water and saturated aqueous sodium chloride solution, dried over sodium sulfate, and then concentrated under reduced pressure. The residue was purified by column chromatography over silica (ethyl acetate:n-hexane=1:1) to obtain Compound 18 (32 mg, 32%) as yellow solid.
mp 190-192°C
¹H NMR(CDCl₃) δ 3. 87(s, 3H), 6. 79(t, J=8. 0Hz, 1H), 6. 87(t, J=8. 0Hz, 1H), 6. 94( d, J=8. 0Hz, 2H), 7. 14(t, J=8. 0Hz, 1H), 7. 18(t, J=8. 0Hz, 1H), 7. 30(d, J=8. 0Hz, 1H) , 7. 47(d, J=8. 0Hz, 1H), 7. 72(brs, 1H), 7. 81(s, 1H), 8. 08(s, 1H).
IR(KBr) 3450, 1760, 1700, 1640, 1340, 1120, 745cm⁻¹.
MS m/z 342(M⁺)

### Referential Example 17

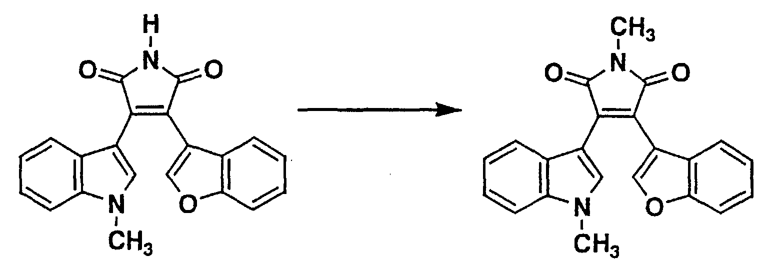

To a solution of Compound 18 (30 mg, 0.09 mmol) in DMF (0.5 mL) was added sodium hydride (60 to 72%, oily, 5.3 mg), and the whole was stirred for 10 minutes. Methyl iodide (8.2 µL, 0.13 mmol) was added, and the mixture was stirred at room temperature for 1 hour. Saturated aqueous sodium chloride solution was added and the mixture was extracted with ethyl acetate. The extract was washed with saturated aqueous sodium chloride solution, dried over sodium sulfate, and concentrated under reduced pressure. The residue was purified by column chromatography over silica gel (ethyl acetate:n-hexane=1:1) to obtain Compound 19 (27 mg, 84%) as red solid.
mp 161-165°C
¹H NMR(CDCl₃) δ 3. 19(s, 3H), 3. 87(s, 3H), 6. 78(t, J=8. 2Hz, 1H), 6. 86(t, J=8. 2Hz, 1H), 6. 93(d, J=8. 2Hz, 1H), 6. 95(d, J=8. 2Hz, 1H), 7. 12(t, J=8. 2Hz, 1H), 7. 17( t, J= 8. 2Hz, 1H), 7. 29(d, J=8. 2Hz, 1H), 7. 47(d, J=8. 2Hz, 1H), 7. 79(s, 1H), 8. 08(s, 1H).
IR(KBr) 3440, 1760, 1695, 1640, 1450, 1370, 1120, 740cm⁻¹.
MS m/z 356(M⁺)

### Referential Example 18

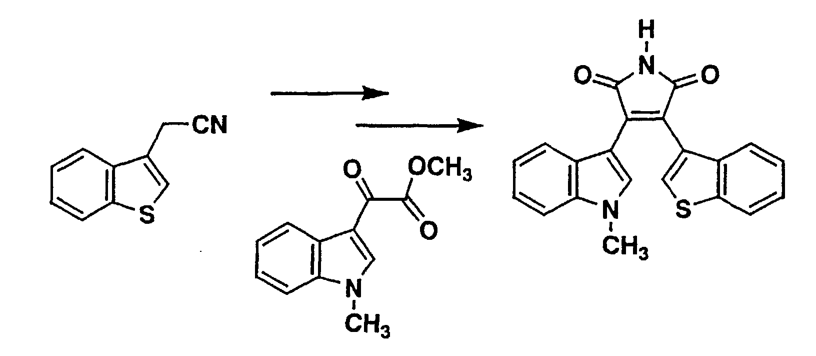

To a solution of benzothiophene-3-acetonitrile (300 mg, 1.73 mmol) synthesized according to a known method (*J*. *Med. Chem.,* Vol. 35, p. 1176, 1992) in tert-butyl alcohol (4 mL) was added potassium hydride (780 mg, 0.014 mol), and the whole was refluxed for 1.5 hours. After cooling to room temperature and acidification with 1N hydrochloric acid, resulting solid was filtrated, washed with water and diethyl ether gave benzothiofuran-3-acetamide (222 mg, 67%) as colorless solid.
mp 170-173°C
¹H NMR (DMSO-d₆) δ 3. 66(s, 2H), 6. 97(brs, 1H), 7. 36(t, J=7. 4Hz, 1H), 7. 40(t, J= 7. 4Hz. 1H), 7. 50(s, 1H), 7. 55(brs, 1H), 7. 84(d, J=7. 4Hz, 1H), 7. 97(d, J=7. 4Hz, 1H).
IR(KBr) 3390, 3200, 1660, 1630, 1420, 1400, 1280, 780, 760, 740, 700, 600cm⁻¹.
MS m/z 191(M⁺)

To a solution of benzothiophene-3-acetamide (50 mg, 0.31 mmol) and methyl (1-methyl-indol-3-yl)glyoxylate (62 mg, 0.29 mmol) synthesized according to a known method (*J*. *Org. Chem.,* Vol. 63, p. 6053, 1998) in DMF (1 mL) was added a solution of potassium tert-butoxide (65 mg, 0.58 mmol) in DMF (1.5 mL), and the whole was stirred at 45°C for 6 hours. After cooling to room temperature and addition of 1N aqueous hydrochloric acid, the mixture was extracted with ethyl acetate. The extract was washed with water and saturated aqueous sodium chloride solution, dried over sodium sulfate, and then concentrated under reduced pressure. The residue was purified by column chromatography over silica (ethyl acetate:n-hexane=1:4) to obtain Compound 20 (48 mg, 51%) as yellow solid.
mp 269-272°C
¹H NMR(DMSO-d₆) δ 3. 86(s, 3H), 6. 35(d, J=7. 9Hz, 1H), 6. 54(t, J=7. 9Hz, 1H), 7. 0 0(t, J=7. 9Hz, 1H), 7. 10(t, J=7. 9Hz, 1H), 7. 26(t, J=7. 9Hz, 1H), 7. 38(d, J=7. 9Hz, 1 H), 7. 39(d, J=7. 9Hz, 1H), 7. 87(s, 1H), 7. 98(d, J=7. 9Hz, 1H), 8. 09(s, 1H), 11. 14(s , 1H).
IR(KBr) 3450, 1760, 1700, 1620, 1520, 1340, 1240, 750cm⁻¹.
MS m/z 358(M⁺)

Referential Example 19

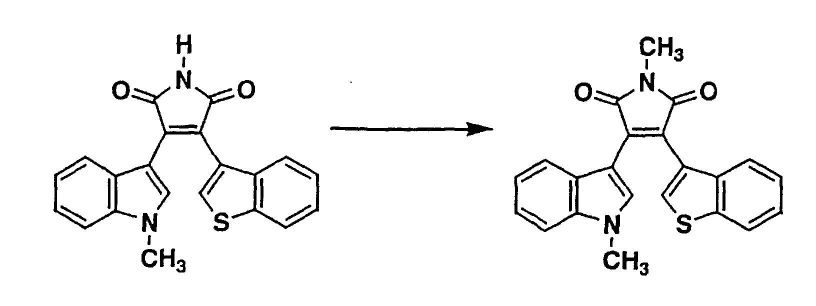

To a solution of Compound 20 (30 mg, 0.08 mmol) in DMF (0.5 mL) was added sodium hydride (60 to 72%, oily, 5 mg), and the whole was stirred for 10 minutes. Methyl iodide (7.8 µL, 0.13 mmol) was added, and the mixture was stirred at room temperature for 1 hour. Saturated aqueous sodium chloride solution was added, and the mixture was extracted with ethyl acetate. The extract was washed with saturated aqueous sodium chloride solution, dried over sodium sulfate, and concentrated under reduced pressure. The residue was purified by column chromatography over silica gel (ethyl acetate:n-hexane=1:1) to obtain Compound 21 (28 mg, 92%) as red solid.
mp 200-204°C
¹H NMR(CDCl₃) δ 3. 20(s, 3H), 3. 82(s, 3H), 6. 45(d, J=8. 0Hz, 1H), 6. 62(t, J=8. 0Hz, 1H), 7. 04(t, J=8. 0Hz, 2H), 7. 20(d, J=8. 0Hz, 1H), 7. 21(t, J=8. 0Hz, 1H), 7. 40(d, J= 8. 0Hz, 1H), 7. 68(s, 1H), 7. 81 (d, J= 8. 0Hz, 1H), 7. 96(s, 1H).
IR(KBr) 3450, 1760, 1700, 1630, 1520, 1450, 1435, 1390, 1380, 1240, 745cm⁻¹.
MS m/z 372(M⁺)

### Industrial Applicability

Since the indolylmaleimide derivatives according to the present invention inhibit cell death, they are considered to be useful for prevention or treatment of all the diseases whose onset and exacerbation are associated with cell death. Accordingly, the derivatives have uses as remedies for neurodegenerative diseases such as Alzheimer's disease, spinal muscular atrophy, amyotrophic lateral screrosis, Parkinson's disease, Huntington's disease, pigmentary degeneration of the retina, glaucoma, cerebellar degeneration and neonatal jaundice; myasthenia gravis; brain ischemia from apoplexy and the like, and successive delayed neuronal death, ischemic heart disease due to myocardial infarction (myocardial ischemia and disorder after reperfusion); viral myocarditis; autoimmune myocarditis (congestive cardiomyopathy and chronic myocarditis); myocardial disorders or death due to hypertrophic heart and heart failure; arrythmogenic right ventricular cardiomyopathy; alcoholic hepatitis and viral hepatitis; renal diseases such as glomerulonephritis, hemolytic uremic syndrome and the like; acquired immunodeficiency syndrome (AIDS); inflammatory skin disorders such as toxic epidermal necrolysis (TEN) and multiform exudative erythema; alopecia; graft versus host disease (GVH); radiation disorders; disorders due to toxic agents including side effects due to anti-cancer drugs, anti-viral drugs and the like; sepsis; osteomyelo-dysplasia such as aplastic anemia and the like; insulin dependent diabetes; prion diseases such as Creutzfeldt-Jakob's disease and the like; and functional deficiency of transplanted organs and the like, or uses as drugs for stopping or inhibiting progress and exacerbation of the diseases; and also uses as preservatives for organs, tissues and cells.

## Claims

1. A cell death inhibitor comprising, as an active ingredient, an indolylmaleimide derivative represented by the following formula (I): 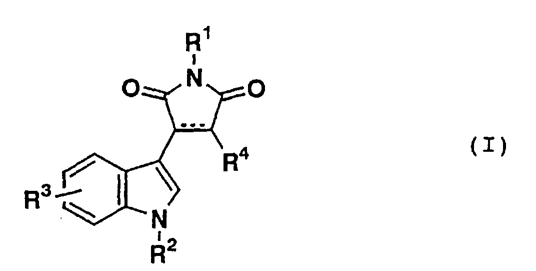 wherein R¹ represents an alkyl group which may be substituted, an alkenyl group which may be substituted, an aryl group which may be substituted, hydroxyl group, an alkoxyl group which may be substituted, an aryloxy group which may be substituted, an amino group which may be substituted, or hydrogen atom; R² represents hydrogen atom, an alkyl group which may be substituted, an alkenyl group which may be substituted, an alkynyl group which may be substituted, an aryl group which may be substituted, an acyl group which may be substituted, an alkoxy- or aryloxycarbonyl group which may be substituted, an alkyl-or arylthiocarbonyl group which may be substituted, an aminocarbonyl group which may be substituted, an alkyl- or arylsulfonyl group which may be substituted, an alkoxyl group which may be substituted, an aryloxy group which may be substituted, or hydroxyl group; R³ represents substituent(s) on an indole ring, and represents, number and position (2-, 4-, 5-, 6-, or 7-position as position number of the indole ring) of the substituent(s) and kinds of the substituent(s) may be the same or different, hydrogen atom, an alkyl group which may be substituted, an alkenyl group which may be substituted, an alkynyl group which may be substituted, an aryl group which may be substituted, an acyl group which may be substituted, an alkoxy- or aryloxycarbonyl group which may be substituted, an alkoxy- or aryloxycarbonyloxy group which may be substituted, an alkyl- or arylthiocarbonyl group which may be substituted, an aminocarbonyl group which may be substituted, an alkyl- or arylsulfonyl group which may be substituted, an alkoxyl group which may be substituted, an aryloxy group which may be substituted, an alkyl- or arylthio group which may be substituted, hydroxyl group, carboxyl group, cyano group, nitro group, an amino group which may be substituted, or a halogen atom; R⁴ represents hydrogen atom, an alkyl group which may be substituted, an alkenyl group which may be substituted, an alkynyl group which may be substituted, an aryl group which may be substituted (except 3-indolyl group), an acyl group which may be substituted, an alkoxy- or aryloxycarbonyl group which may be substituted, an alkyl- or arylthiocarbonyl group which may be substituted, an aminocarbonyl group which may be substituted, an alkyl- or arylsulfonyl group which may be substituted, an alkoxyl group which may be substituted, an aryloxy group which may be substituted, alkyl- or arylthio group which may be substituted, hydroxyl group, carboxyl group, cyano group, nitro group, or amino group which may be substituted; R² and R³, R² and R⁴, R³ and R⁴ may be combined to form a hydrocarbon chain which may be substituted; and in the formula, the bond accompanying a dotted line represents a double bond or a single bond, or a pharmaceutically acceptable salt thereof.

2. A drug for treating or preventing progress of symptoms, through inhibiting death of neurons, of neurodegenerative diseases such as Alzheimer's disease, spinal muscular artophy (SMA), amyotrophic lateral screrosis (ALS), Parkinson's disease, Huntington's disease, pigmentary degeneration of the retina, glaucoma and cerebellar degeneration, comprising a indolylmaleimide derivative represented by the above formula (I) or a pharmaceutically acceptable salt thereof as an active ingredient.

3. A drug for treating or preventing progress of symptoms, through inhibiting death of neurons, of neonatal jaundice, comprising a indolylmaleimide derivative represented by the above formula (I) or a pharmaceutically acceptable salt thereof as an active ingredient.

4. A drug for treating or preventing progress of symptoms, through inhibiting cell death, of myasthenia gravis, comprising a indolylmaleimide derivative represented by the above formula (I) or a pharmaceutically acceptable salt thereof as an active ingredient.

5. A drug for treating or preventing progress of symptoms, through inhibiting death of neurons, of brain ischemia and delayed neuronal death (DND), comprising a indolylmaleimide derivative represented by the above formula (I) or a pharmaceutically acceptable salt thereof as an active ingredient.

6. A drug for treating or preventing progress of symptoms, through inhibiting death of myocardial cells, of ischemic heart disease, viral myocarditis, autoimmune myocarditis, myocardial disorders/cell death due to hypertrophic heart and heart failure, or arrythmogenic right ventricular cardiomyopathy, comprising a indolylmaleimide derivative represented by the above formula (I) or a pharmaceutically acceptable salt thereof as an active ingredient.

7. A drug for treating or preventing progress of symptoms, through inhibiting death of hepatic cells, of alcoholic hepatitis or viral hepatitis, comprising a indolylmaleimide derivative represented by the above formula (I) or a pharmaceutically acceptable salt thereof as an active ingredient.

8. A drug for treating or preventing progress of symptoms, through inhibiting death of renal cells, of renal diseases, comprising a indolylmaleimide derivative represented by the above formula (I) or a pharmaceutically acceptable salt thereof as an active ingredient.

9. A drug for treating or preventing progress of symptoms, through inhibiting excessive death of T-cells, of acquired immunodeficiency syndrome (AIDS), comprising a indolylmaleimide derivative represented by the above formula (I) or a pharmaceutically acceptable salt thereof as an active ingredient.

10. A drug for treating or preventing progress of symptoms, through inhibiting cell death, of inflammatory skin disorders, alopecia, or graft versus host disease (GVH), comprising a indolylmaleimide derivative represented by the above formula (I) or a pharmaceutically acceptable salt thereof as an active ingredient.

11. A drug for treating or preventing disorders or side effects, through inhibiting cell death, of disorders due to radiation or drugs, comprising a indolylmaleimide derivative represented by the above formula (I) or a pharmaceutically acceptable salt thereof as an active ingredient.

12. A drug for treating or preventing progress of symptoms, through inhibiting cell death, of sepsis, comprising a indolylmaleimide derivative represented by the above formula (I) or a pharmaceutically acceptable salt thereof as an active ingredient.

13. A drug for treating or preventing progress of symptoms, through inhibiting death of cells derived from bone marrow, of osteomyelo-dysplasia, comprising a indolylmaleimide derivative represented by the above formula (I) or a pharmaceutically acceptable salt thereof as an active ingredient.

14. A drug for treating or preventing progress of symptoms, through inhibiting cell death, of insulin dependent diabetes, comprising a indolylmaleimide derivative represented by the above formula [II] or a pharmaceutically acceptable salt thereof as an active ingredient.

15. A drug for treating or preventing progress of symptoms, through inhibiting death of neurons, of prion diseases, comprising a indolylmaleimide derivative represented by the above formula (I) or a pharmaceutically acceptable salt thereof as an active ingredient.

16. A drug for treating or preventing functional deficiency of transplanted organs, tissues or cells at transplantation of organs, tissues or cells, comprising a indolylmaleimide derivative represented by the above formula (I) or a pharmaceutically acceptable salt thereof as an active ingredient.

17. A preservative for organs, tissues or cells, comprising a indolylmaleimide derivative represented by the above formula (I) or a pharmaceutically acceptable salt thereof as an active ingredient.
